# EUROPÄISCHE PATENTSCHRIFT

(11) **EP 1 148 060 B1**
(45) Veröffentlichungstag und Bekanntmachung des Hinweises auf die Patenterteilung: **10.09.2003**
(21) Anmeldenummer: 01107896.1
(22) Anmeldetag: 11.04.2001
(51) Int. Cl.: C07F 9/38, A61K 6/00, C08F 30/02, C07F 9/6509

(54) **Hydrolysestabile und polymerisierbare Acrylphosphonsäure**
Hydrolysis-stable and polymerisable acrylphosphonic acids
Acides acrylphosphoniques stables à l'hydrolyse et polymérisables

(30) Priorität: 17.04.2000 DE 10018968
(43) Veröffentlichungstag der Anmeldung: 24.10.2001
(73) Patentinhaber: Ivoclar Vivadent AG, 9494 Schaan (LI)
(72) Erfinder: Moszner, Norbert, 9492 Eschen (LI); Rumphorst, André, 9490 Vaduz (LI); Rheinberger, Volker, 9490 Vaduz (LI); Zeuner, Frank, 9490 Vaduz (LI)
(74) Vertreter: UEXKÜLL & STOLBERG

(56) Entgegenhaltungen:
- EP-A- 0 909 761
- US-A- 4 650 847

## Beschreibung

Die vorliegende Erfindung betrifft polymerisierbare Acrylphosphonsäuren, die eine hohe Hydrolysestabilität aufweisen und sich insbesondere zur Herstellung oder als Bestandteile von Polymeren, Adhäsiven oder anderen Materialien und hauptsächlich von Dentalmaterialien eignen.

Polymerisierbare Phosphonsäuren sind vor allem als Comonomere von polymer-chemischer Bedeutung. Sie gestatten die Herstellung von organischen Polymeren mit hoher thermischer Stabilität, guten Hafteigenschaften, schwerer Entflammbarkeit und guter Löslichkeit in polaren Lösungsmitteln. Für diesen Zweck sind zahlreiche monomere Phosphonsäuren mit polymerisierbaren Vinyl-, Dienyl-, Allyl- oder Styrylgruppen synthetisiert und polymerisiert worden. Eine Übersicht zu Phosphonsäuren gibt Houben-Weyl, Methoden oder Organischen Chemie, Band E 20 (2. Teil), Georg Thieme Verlag, Stuttgart-New York 1987, S. 1300 ff. Beispiele für solche konventionellen polymerisierbaren Phosphonsäuren sind Vinylphosphonsäure, Allylbenzolphosphonsäure, α-Aminoallylphosphonsäure, Phenylethenphosphonsäure, 1,3-Butadien- oder Isoprenphosphonsäure, 4-Vinylbenzolphosphonsäure oder 2-(4-Vinylphenyl)-ethanphosphonsäure.

Phosphonsäuren, bei denen die C=C-Gruppierung direkt oder über ein Sauerstoffatom an das Phosphoratom gebunden ist, wie z.B. Vinylphosphonsäure oder Ethylphosphonsäuremonovinylester, zeigen allerdings nur eine mäßige Neigung zur Homopolymerisation, so daß nur Homopolymere mit einer geringen Molmasse zugänglich sind.

Hochmolekulare Polymerisate können demgegenüber von (Meth)acrylphosphonsäuren oder -estern erhalten werden, bei denen die (Meth)acrylgruppe nicht direkt sondern über eine hydrolysestabile Spacergruppe an den Phosphor gebunden ist. Derartige (Meth)acrylphosphonsäure-Derivate werden beispielsweise in der DE-B-27 11 234 beschrieben.

Die DE-A-32 10 775 offenbart 2-Acrylamido-2-methyl-propanphosphonsäure mit der Formel CH₂=CH-CONH-C(CH₃)₂-CH₂-P(=O)(OH)₂ sowie deren Verwendung zur Herstellung von Copolymerisaten.

Die DE-A-33 13 819 und die JP 62-63314 (Chem. Abstr. 107 (1987), 41318f) offenbaren Methacrylsäure-(2-phosphono-1,1-dimethylethylamin) der Formel CH₂=C(CH₃)-CONH-C(CH₃)₂-CH₂-P(=O)(OH)₂.

Gemäß EP-B-0 089 654 und US-A-4 650 591 soll sich Acrylsäure-(2-phosphono-1,1-dimethylethylamin), auch als 2-Acrylamido-2-methylpropanphosphonsäure bezeichnet, in Form ihrer Homo- oder Copolymeren als Korrosionsinhibitor eignen.

Die DD-A-273 846 offenbart Haftvermittler auf der Basis von N-Acyl-aminomethan-bisphosphonsäurederivaten.

Diese bekannten (Meth)acrylphosphonsäure-Derivate sind in wäßriger Lösung nicht stabil. Vielmehr findet bei ihnen eine hydrolytische Abspaltung der (Meth)acrylgruppe statt, die durch dissoziierte Protonen der Phosphonsäuregruppe sogar noch katalysiert und damit beschleunigt wird.

Bei einer ganzen Reihe von Anwendungen polymerisierbarer Phosphonsäuren ist jedoch der Einsatz von wäßrigen Lösungen von Vorteil oder zwingend notwendig. Dies ist z.B. bei der Herstellung von niedrigviskosen Adhäsiven, die frei von organischen Lösungsmitteln sind, oder bei dentalen Adhäsiven der Fall, die nur in wäßriger Form zu einer optimalen Benetzung der feuchten Dentinoberfläche führen.

Die DE 197 46 708 A1 offenbart polymerisierbare Acrylphosphonsäuren, die in wäßriger Lösung hydrolysestabil sind, über gute Hafteigenschaften verfügen, mit herkömmlichen radikalischen Initiatoren polymerisiert werden können und sich daher als Bestandteil insbesondere von Adhäsiven, Formkörpern, Zementen oder Kompositen und vor allem von Dentalmaterialien eignen. Die Acrylphosphonsäuren zeigen in Form ihrer Carbonsäureester eine gute Löslichkeit in Wasser und polaren organischen Lösungsmitteln während sie in Form der Carbonsäuren zwar gut in Wasser aber kaum in organischen Lösungsmitteln löslich sind. Das unterschiedliche Lösungsverhalten von Ester und Säure kann bei wasserhaltigen Materialien nachteilig sein. Die Hydrolyse der Carbonsäureester zur freien Carbonsäure unter Abspaltung von Alkohol kann die Löslichkeit der Monomeren signifikant verändern und so zur partiellen oder vollständigen Ausfällung der Phosphonsäurekomponente führen und damit die Eigenschaften des Materials beeinflussen.

Aufgabe der Erfindung ist die Bereitstellung von polymerisationsfähigen Acrylphosphonsäuren, die in Gegenwart von Wasser bei Raumtemperatur praktisch vollkommen hydrolysestabil sind.

Diese Aufgabe konnte überraschend durch Acrylphosphonsäuren der folgenden allgemeinen Formel (I) gelöst werden 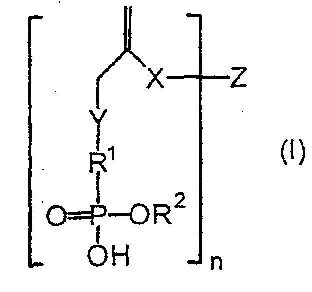 worin R¹, R², R³, X, Y, Z und n die folgenden Bedeutungen haben:
- R¹ =: ein geradkettiger oder verzweigter C₁- bis C₁₀-Alkylenoder C₆- bis C₁₄-Arylen-Rest;
- R² =: Wasserstoff, ein geradkettiger oder verzweigter C₁- bis C₁₀-Alkyl- oder C₆- bis C₁₀-Arylrest;
- Y =: Sauerstoff, Schwefel, C₁- bis C₈-Alkylen oder entfällt;
- n =: 1, 2, 3, 4, oder 5;
wobei
- X =: CN, n = 1 und Z = entfällt oder
- X =: CONR³ mit
R³ = Wasserstoff, ein geradkettiger oder verzweigter C₁- bis C₁₀-Alkylrest oder ein C6- bis C₁₀-Aryl-Rest;
mit der Maßgabe daß
für
- n =: 1
- Z =: Wasserstoff oder ein geradkettiger oder verzweigter C₁bis C₁₀-Alkylrest oder ein Phenylrest; und
für
- n =: 2 bis 5
- Z =: ein n-fach mit der in Klammern stehenden Struktur der Formel (I) substituierter, aliphatischer, aromatischer oder araliphatischer, geradkettiger oder verzweigter Kohlenwasserstoffrest mit 1 bis 14 Kohlenstoffatomen, wobei Z und R³ auch Teil eines gemeinsamen Ringes sein können, und
wobei die einzelnen Reste substituiert oder unsubstituiert sein können.

Die einzelnen Alkyl-, Aryl-, Alkylen-, Arylen-, Phenyl-, Phenylen- und Arylenalkylenreste können durch einen oder mehrere Substituenten, wie Cl, Br, CH₃O, OH, COOH, CN, =O, =S, =NR² oder -NR³-CO-C(=CH₂)CH₂-Y-R¹-PO(OH)₂ substituiert sein.

Die Nitrile (X = CN) lassen sich in die Amide (X = CONR³) überführen und können daher als deren Vorstufen angesehen werden.

Weiter existieren für die oben angegebenen Variablen der Formel (I) bevorzugte Definitionen, die, sofern nicht anders angegeben, unabhängig voneinander gewählt werden können und wie folgt sind:
- R¹ =: ein geradkettiger oder verzweigter C₁- bis C₅-Alkylenrest oder Phenylen;
- R² =: Wasserstoff oder ein geradkettiger C₁- bis C₃-Alkylrest;
- Y =: Sauerstoff oder entfällt;
- X =: CN oder CONR³ mit
R³ = Wasserstoff, ein geradkettiger C₁- bis C₆-Alkylrest, ein Phenylrest oder zusammen mit Z Teil eines sechsgliedrigen Rings;
- n =: 1 oder 2;
- Z =: Wasserstoff oder-ein geradkettiger oder verzweigter C₁bis C₁₀-Alkylrest, ein Phenylrest oder zusammen mit R³ Teil eines sechsgliedrigen Rings (für n = 1); und
- Z =: ein geradkettiger C₁- bis C₁₀-Alkylenrest oder zusammen mit R³ Teil eines sechsgliedrigen Rings (für n ≥ 2).

Besonders bevorzugte Bedeutungen, die ebenfalls unabhängig voneinander gewählt werden können, sind:
- R¹ =: ein geradkettiger oder verzweigter C₁- bis C₄-Alkylenrest;
- R² =: Wasserstoff oder ein Methylrest;
- Y =: Sauerstoff;
- X =: CONR³;
- R³ =: Wasserstoff oder ein geradkettiger C₁- bis C₅-Alkylrest;
- Z =: Wasserstoff oder ein geradkettiger C₁- bis C₆-Alkylrest (für n = 1); und
- Z =: ein geradkettiger C₁- bis C₅-Alkylenrest (für n ≥ 2).

Die Reste R¹, R², R³ und/oder Y sind vorzugsweise unsubstituiert, der Rest Z ist vorzugsweise unsubstituiert oder durch =O, =S, =NR² oder -NR³-CO-C(=CH₂)CH₂-Y-R¹-PO(OH)₂ substituiert.

Bevorzugte Verbindungen sind demgemäß solche, bei denen mindestens eine besonders bevorzugt alle der Variablen der Formel (I) die vorstehend beschriebenen bevorzugten Definition aufweisen, wobei die Formel (I) alle durch die genannten Substituenten möglichen Stereoisomere und ihre Mischungen, wie Racemate, einschließt.

Die erfindungsgemäßen Acrylphosphonsäuren der Formel (I) (X = CN, Z entfällt) lassen sich durch Umsetzung von am Alkylrest Y-funktionalisierten Alkylphosphonsäureestern APE (R² = Alkyl) mit α-Halogenmethylacrylnitrilen (U = Halogen, vor allem Cl oder Br) HMAN und nachfolgende Abspaltung der Alkylgruppen R² unter Anwendung der aus der organischen Chemie bekannten Methoden für die Knüpfung von C-C-, C-O- oder -C-S-Bindungen (vgl. C. Weygand, G. Hilgetag, Organisch-chemische Experimentierkunst, Johann Ambrosius Bart Verlag, Leipzig 1970, S. 963f., 362f. und 657f.) herstellen. Dabei wird für die beiden Phosphonsäure-Gruppen die Schutzgruppen-Technik eingesetzt, d.h. daß die Umsetzungen z.B. mit den entsprechenden Phosphonsäureestern durchgeführt werden, aus denen dann anschließend in Abhängigkeit vom eingesetzten Hydrolyse-Reagenz die Mono- (R² = Alkyl) oder Dihydrogenphosphonsäuren (R² = H) der Formel (I) freigesetzt werden:

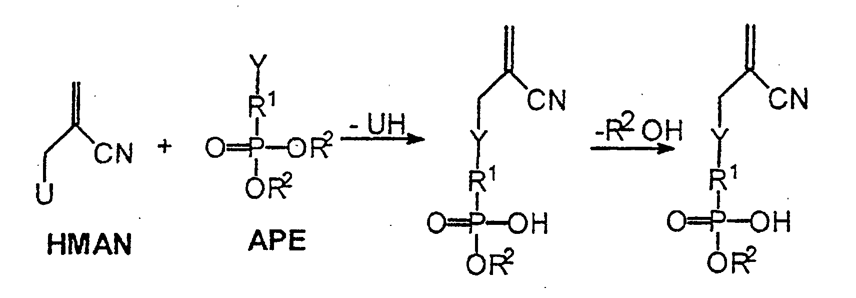

Konkret führt die Umsetzung von α-Chlormethylacrylnitril mit 2-Hydroxyethylphosphonsäuredimethylester über 2-[4-(Dimethoxyphosphoryl)-2-oxybutyl]-acrylnitril nach Silylierung mit Trimethylsilylbromid und Desilylierung mit Methanol zur entsprechenden Phosphonsäure {2-[4-(Dihydroxyphosphoryl)-2-oxabutyl]-acrylnitril}:

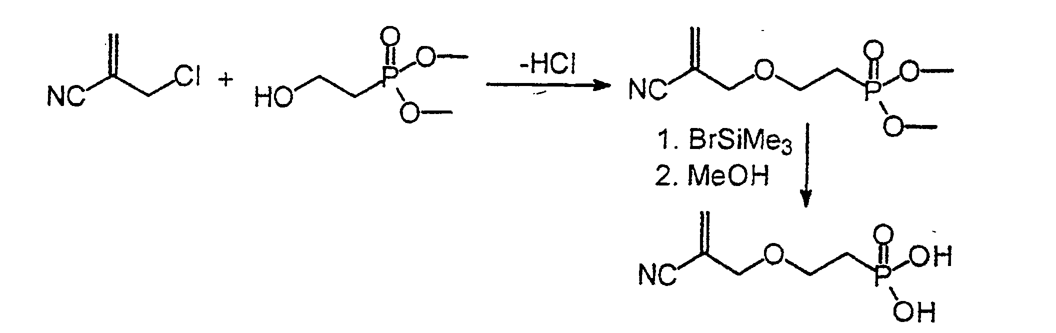

Die α-Halogenmethylacrylnitrile HMAN sind durch Reaktion von Acrylnitril mit Formaldehyd in Gegenwart von 1,4-Diazabicyclo[2.2.2]octan (DABCO) und nachfolgende Halogenierung mit anorganischen Säurechloriden, wie SOCl₂, PCl₃ oder PBr₃ zugänglich (vgl. DE-OS 34 44 098 und G.F. Meijs, E. Rizzardo, S.H. Thang, Polym. Bull. 24 (1990) 501).

Beispielsweise führt die Umsetzung von Acrylnitril mit Formaldehyd über α-Hydroxymethylacrylnitril -nach Chlorierung mit Thionylchlorid zum α-Chlormethylacrylnitril:

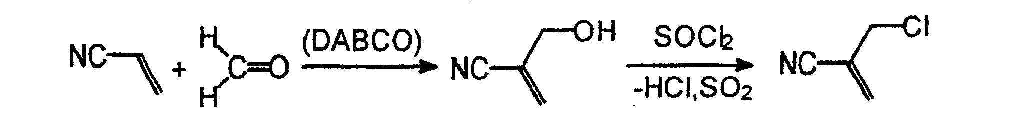

Geeignete Phosphonsäureester APE können auf unterschiedlichen Wegen erhalten werden. Eine besonders geeignete Reaktion für die Herstellung von Alkanphosphonsäureestern ist die Michaelis-Arbusow-Reaktion (vgl. G.M. Kosolapoff, Org. Reactions 6 (1951) 273), bei der Trialkylphosphite, z.B. Triethylphosphit, und Halogenalkane miteinander umgesetzt werden, z.B.:

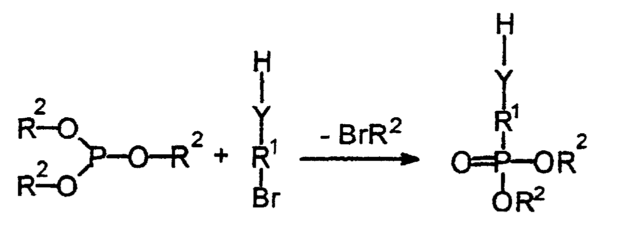

Konkret kommt es bei der Reaktion von Triethylphosphit mit 2-Bromethanol zur Bildung des 2-Hydroxyethylphosphonsäurediethylesters:

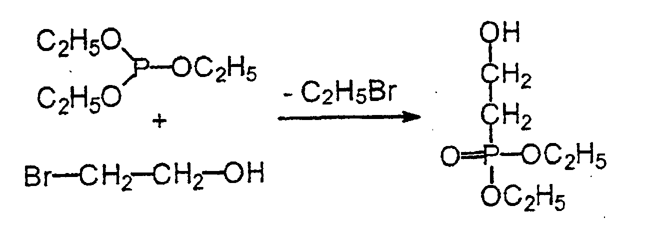

Dabei muß gegebenenfalls auch der Y-Substituent geschützt werden. Eine weitere Möglichkeit der Synthese von Hydroxyalkylphosphonsäureestern (YH = OH) besteht in der basenkatalysierten Anlagerung von Dialkylphosphiten an mono- oder difunktionelle Aldehyde oder Ketone (F. Texier-Boullet, A. Foucaud, Synthesis, 1982, 916):

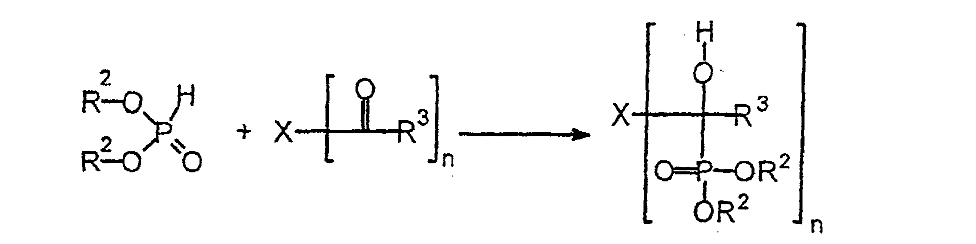

Konkret erhält man durch Umsetzung von Diethylphosphit mit Benzaldehyd den (1-Hydroxy-1-phenyl)-methylphosphonsäurediethylester:

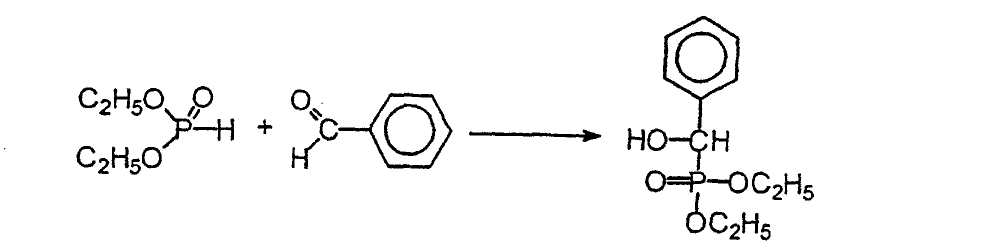

Die hydrolytische Abspaltung der Schutzgruppe erfolgt dann vorzugsweise durch Silylierung mit Trialkylsilylhalogeniden, z.B. Trimethylsilylchlorid/(NaI oder NaBr), und nachfolgende Umsetzung mit Alkoholen oder Wasser (S. Freeman, J. Chem. Soc., Perkin Trans. 2, 1991, 263).

Die erfindungsgemäßen Acrylphosphonsäuren AP der Formel (I) (X = CONR³, n = 1) lassen sich durch Umsetzung von Dialkoxyphosphoryl-acrylsäuren DPA mit monofunktionellen Aminen in Gegenwart eines geeigneten Kondensationsmittels und nachfolgende Hydrolyse der Phosphonsäureestergruppen herstellen.

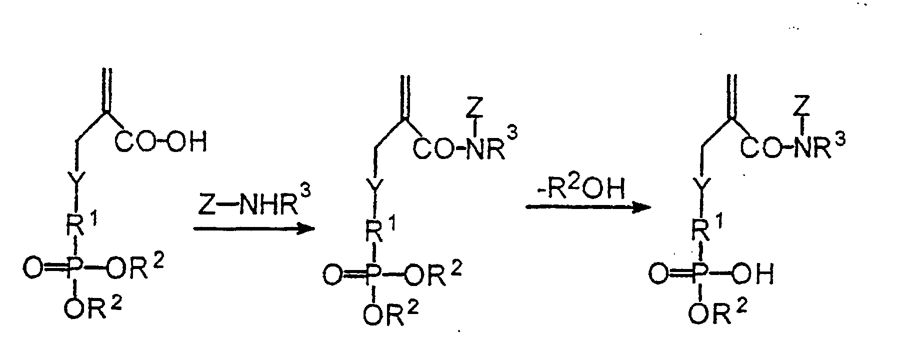

Als Kondensationsmittel für die Amidierung können Carbodiimide bzw. Phosphoroxychlorid (Houben-Weyl, Bd. 15/2, Peptide; 4. Auflage, Georg Thieme Verlag, Stuttgart 1974, S. 103ff. und 232ff.) eingesetzt werden. Die Abspaltung der Phosphonsäureestergruppen erfolgt mittels Trimethylsilylbromid.

Beispielsweise führt die Umsetzung von 2-[4-(Dimethoxyphosphoryl)-2-oxabutyl]-acrylsäure mit Diethylamin über 2-[4-(Dimethoxyphosphoryl)-2-oxabutyl]-acrylsäurediethylamid zu der entsprechenden Acrylamidophosphonsäure {2-[4-(Dihydroxyphosphoryl)-2-oxabutyl]-acrylsäurediethylamid}:

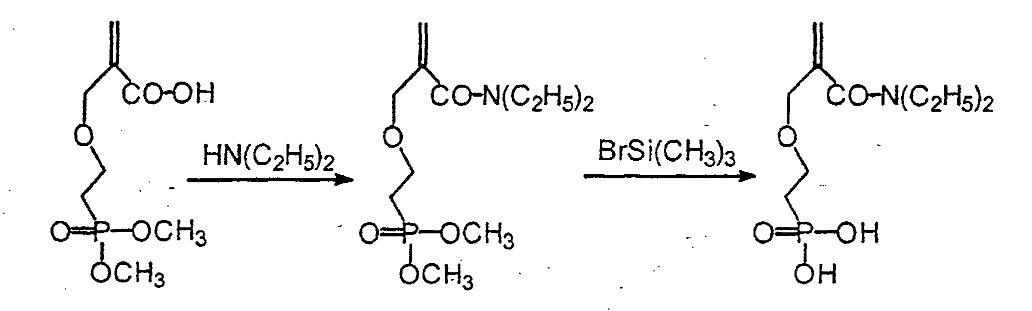

Die eingesetzten Dialkoxyphosphoryl-acrylsäuren DPA lassen sich aus entsprechenden Dialkoxyphosphoryl-acrylsäurealkylestern DPAE (vgl. N. Moszner, F. Zeuner, U.K. Fischer, V. Rheinberger, Macromol. Chem. Phys. 200 (1999) durch selektive alkalische Hydrolyse herstellen, z.B.:

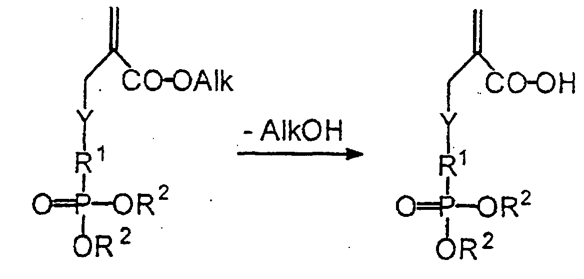

Konkret führt die Umsetzung von 2-[4-(Dimethoxyphosphoryl)-2-oxabutyl]-acrylsäureethylester mit Natriumhydroxid unter Abspaltung von Ethanol zu 2-[4-(Dimethoxyphosphoryl)-2-oxa-butyl]-acrylsäure:

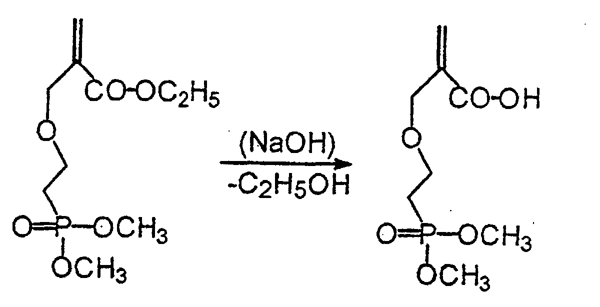

Analog ergibt die Amidierung von Dialkoxyphosphorylacrylsäuren mit Diaminen die erfindungsgemäßen Acrylphosphonsäuren der Formel (I) mit X = CONR³ und n = 2):

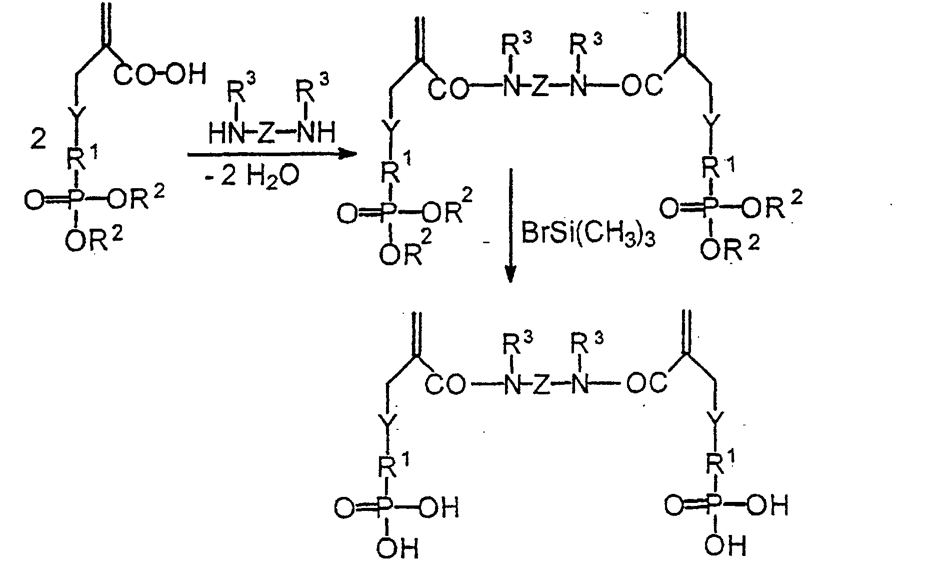

Beispielsweise ergibt die Umsetzung von 2-[4-(Dimethoxyphosphoryl)-2-oxa-butyl]-acrylsäure mit Ethylendiamin das N,N'-Bis-[(6-dimethoxyphosphoryl)-4-oxa-2-methylen-hexanoyl]-ethylendiamin, das sich durch Behandlung mit Trimethylsilylbromid in das N,N'-Bis-[(6-dihydroxyphosphoryl)-4-oxa-2-methylen-hexanoyl]-ethylendiamin überführen läßt:

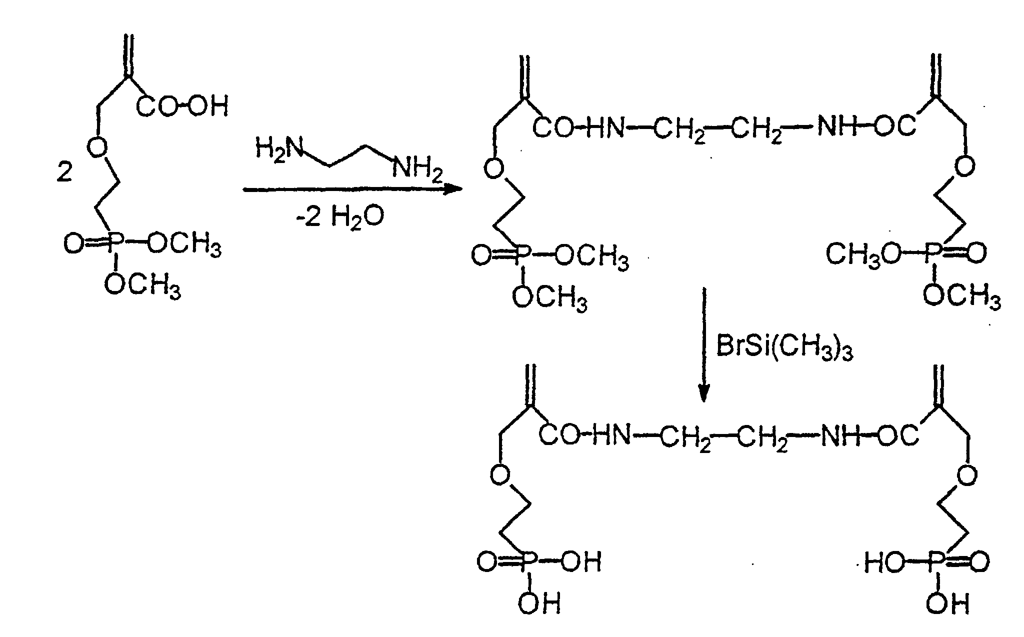

Bevorzugte Beispiele für die erfindungsgemäßen Acrylphosphonsäuren der Formel (I) sind u.a.:

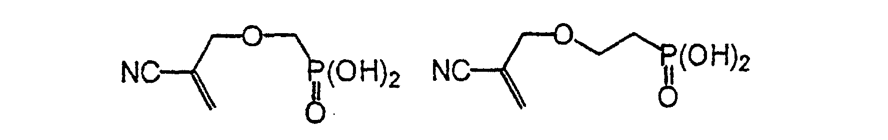

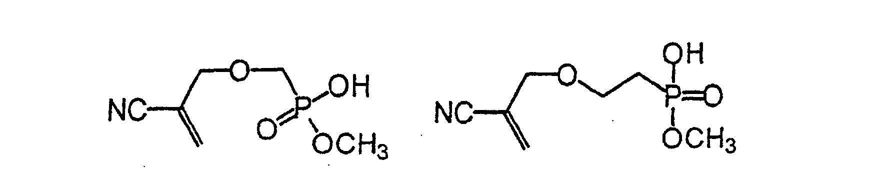

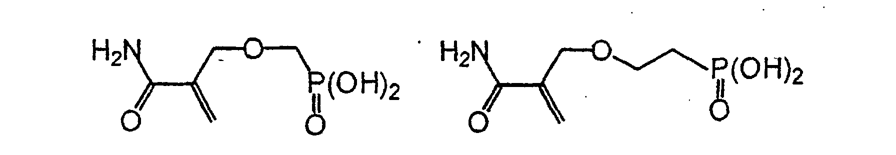

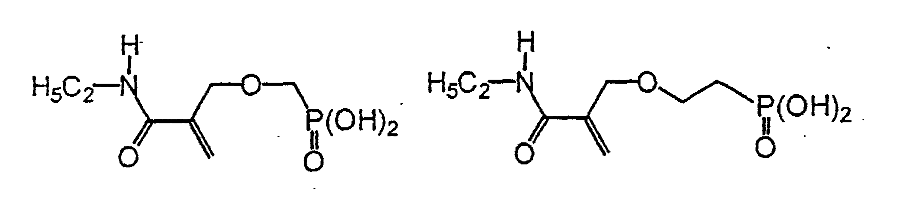

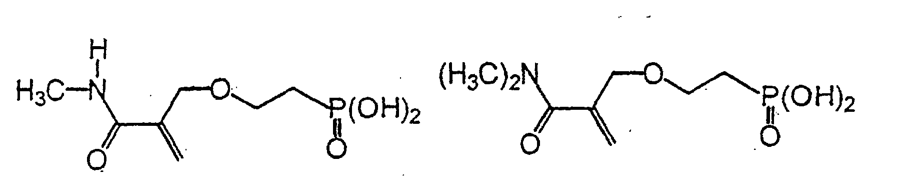

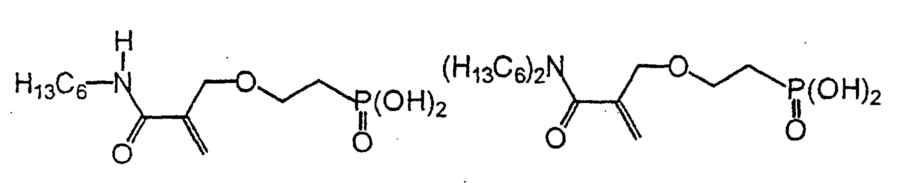

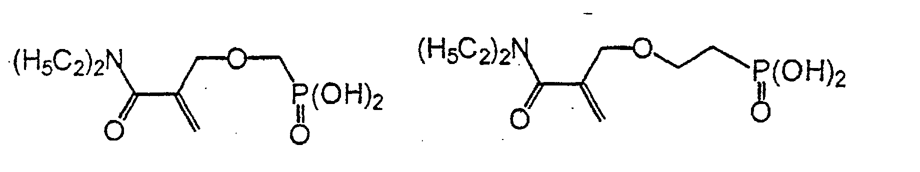

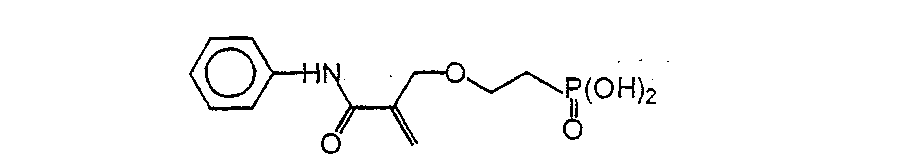

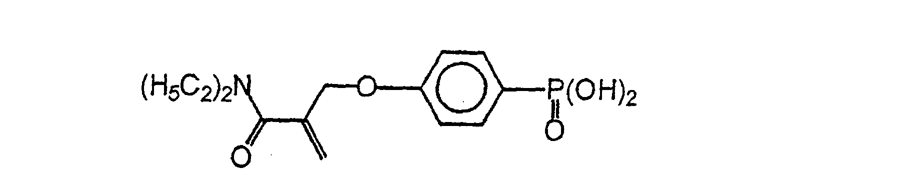

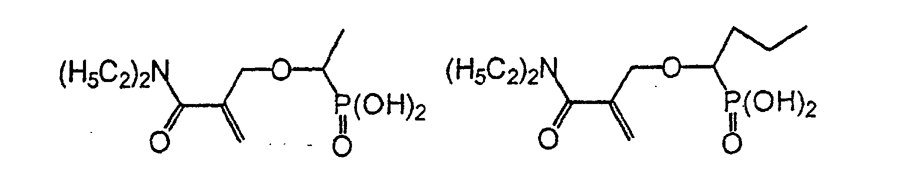

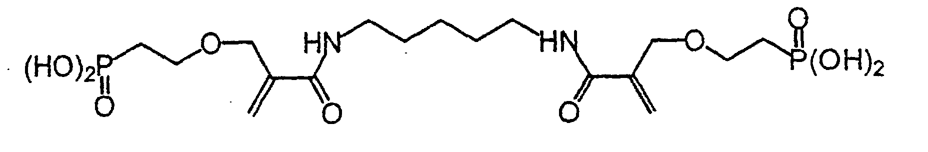

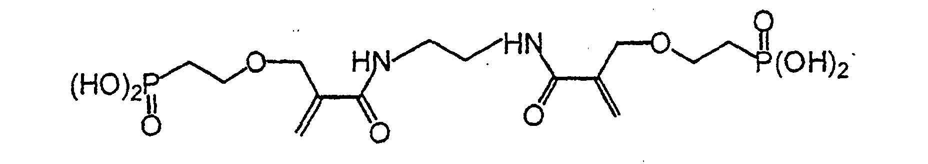

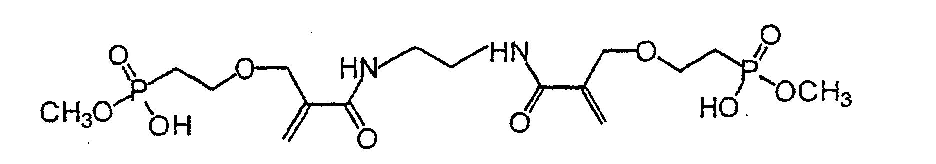

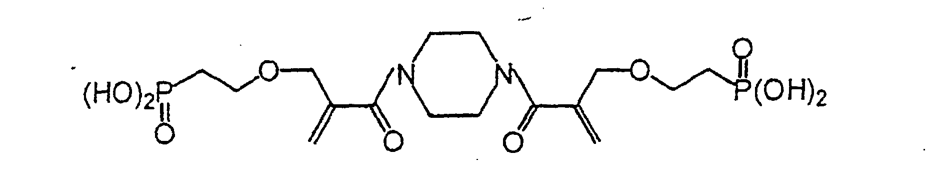

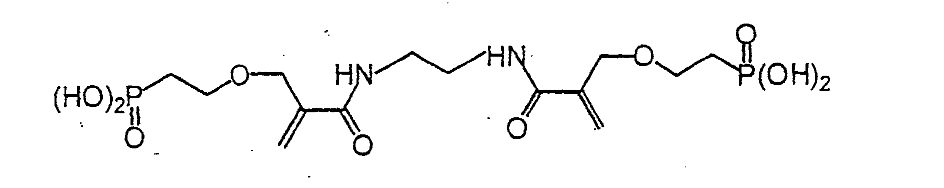

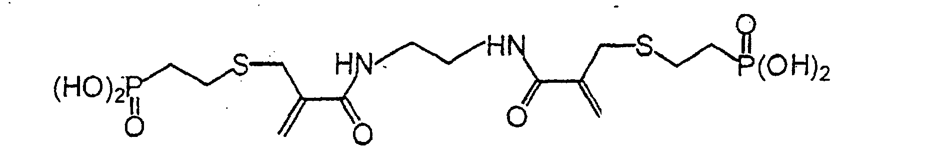

Die erfindungsgemäßen Acrylphosphonsäuren sind bei Raumtemperatur praktisch vollkommen hydrolysestabil. Sie eignen sich daher insbesondere zur Verwendung mit wasserhaltigen Mischungen.

Darüber hinaus zeichnen sich die erfindungsgemäßen Acrylphosphonsäuren im Vergleich zu den entsprechenden Carbonsäurederivaten (X = COO, Z = H) durch eine wesentlich bessere Löslichkeit in polaren organischen Lösungsmitteln wie z.B. Ethanol, Aceton, Methylenchlorid oder Essigsäureethylester aus. Außerdem sind sie gegenüber anderen Verbindungen wie z.B. organischen Lösungsmitteln weitgehend inert, während beispielsweise die entsprechenden Carbonsäureester (X = COO, Z = Alkylrest) in Gegenwart von Ethanol schon bei Raumtemperatur zur Alkoholyse neigen.

Aufgrund des Vorliegens von polymerisierbaren Gruppen eignen sich die erfindungsgemäßen Acrylphosphonsäureester als Ausgangsmaterialien für die Herstellung von Polymeren und Copolymeren. Dabei lassen sie sich mit den bekannten Methoden der radikalischen Polymerisation homopolymerisieren oder z.B. mit geeigneten Comonomeren copolymerisieren.

Zur Durchführung der Polymerisation können die bekannten radikalischen Initiatoren (vgl. Encyclopedia of Polymer Science and Engineering, Vol. 13, Wiley-Interscience Publisher, New York 1988, 754 ff.) eingesetzt werden. Es eignen sich besonders Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich auch Benzpinakol und 2,2'-Dialkylbenzpinakole.

Weiterhin können auch Photoinitiatoren (vgl. J.P. Fouassier, J.F. Rabek (Hrsg.), Radiation Curing in Polymer Science and Technology, Vol. II, Elsevier Applied Science, London und New York 1993) für die Polymerisation mit UV-Licht oder Licht sichtbarer Wellenlängen, wie Benzoinether, Dialkylbenzilketale, Dialkoxyacetophenone, Acylphosphinoxide, α-Diketone, wie 9,10-Phenanthrenchinon, Diacetyl, Furil, Anisil, 4,4'-Dichlorbenzil und 4,4'-Dialkoxybenzil, und Campherchinon, verwendet werden.

Die erfindungsgemäßen Acrylphosphonsäuren können insbesondere als Bestandteil von Adhäsiven, Zementen, Kompositen und Formkörpern sowie bevorzugt von Dentalmaterialien verwendet werden. Die erfindungsgemäßen Verbindungen können dabei auch in polymerisierter oder teilweise polymerisierter Form, d.h. in Form von Polymeren wie Homo- oder Copolymeren, eingesetzt werden, beispielsweise als Komponente von Glasionomerzementen.

Die erfindungsgemäßen Acrylphosphonsäuren lassen sich allein oder in Mischung mit herkömmlichen radikalisch polymerisierbaren Comonomeren, insbesondere mit difunktionellen Vernetzermonomeren polymerisieren. Für die Herstellung von Adhäsiven oder Dentalmaterialien eignen sich vor allem vernetzende bi- oder mehrfunktionelle Acrylate oder Methacrylate, wie z.B. Bisphenol-A-di(meth)acrylat, Bis-GMA (das Additionsprodukt von Methacrylsäure und Bisphenol-A-diglycidylether), UDMA (das Additionsprodukt von Hydroxyethylmethacrylat und 2,2,4-Trimethylhexamethylendiisocyanat), Di-, Tri- oder Tetraethylenglykoldi(meth)acrylat, Trimethylolpropantri(meth)acrylat und Pentaerythrittetra(meth)-acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)-acrylat und 1,12-Dodecandioldi(meth)acrylat.

Die erfindungsgemäßen Acrylphosphonsäuren können in freier Form oder in Form ihrer Salze, d.h. als Phosphonate oder Phosphonatester, eingesetzt werden, wobei im Fall der Salze als Gegenionen vorzugsweise Alkalimetallionen, insbesondere - Natrium- und Lithiumionen, sowie organische Ammoniumionen dienen, insbesondere solche, die sich von Aminbeschleunigern, wie N,N-Dihydroxyethyl-p-toluidin, N,N-Bis-(2-hydroxy-3-methacryloxypropyl-3,5-xylidin oder 4-(Dimethylamino)-benzoesäure-2-ethyl-hexylester, ableiten.

Aminbeschleuniger werden im Dentalbereich als Komponente beispielsweise von Photoinitiatorsystemen eingesetzt. Es handelt sich im allgemeinen um tert. Amine, die als H-Donatoren wirken können und damit die Radikalbildung beschleunigen (vgl. L.A. Linden, "Photocuring of Polymeric Dental Materials and Plastic Composite Resins" in Radiation Curing in Polymer Science and Technology, Vol. IV, J.P. Fouassier, J.F. Rabek (Herausgeber), Elsevier Appl. Sci., London, Ney York 1993, 396f.).

Darüber hinaus können die erfindungsgemäßen Acrylphosphonsäuren oder ihre Mischungen mit anderen radikalisch polymerisierbaren Comonomeren zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂, mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5 µm. Weiterhin können auch röntgenopake Füllstoffe, wie Ytterbiumtrifluorid, oder Glasfasern, Polyamid- oder Kohlenstoff-Fasern eingesetzt werden.

Den Acrylphosphonsäuren oder Mischungen davon können im Bedarfsfall weitere Komponenten zugesetzt werden, vor allem Lösungsmittel, wie Wasser, Methanol, Ethanol, Isopropanol, Methylethylketon, Aceton, Essigsäureethylester, Dimethylformamid, Dimethylsulfoxid oder Mischungen von diesen, sowie Stabilisatoren, UV-Absorber, Farbstoffe, Pigmente oder Gleitmittel. Zur Verwendung in Dentalmaterialien sind als Lösungsmittel Wasser, Ethanol, Aceton und Essigsäureethylester sowie Mischungen davon bevorzugt.

Die erfindungsgemäßen Acrylphosphonsäuren eignen sich besonders als Bestandteil von Dentalmaterialien, wie Befestigungszemente und Füllungskomposite und vor allem Dentaladhäsive. Solche Materialien zeichnen sich durch eine sehr gute Haftung auf unterschiedlichen Substraten, wie der Zahnhartsubstanz und metallischen Substraten, aus und sind unter feuchten Bedingungen hydrolysestabil.

Bevorzugte erfindungsgemäße Dentalmaterialien enthalten die folgenden Komponenten (a), (b), (c), (d) und/oder (e):
(a) 0,5 bis 99 Gew.-%, bevorzugt 10 bis 80 Gew.-% und besonders bevorzugt 20 bis 50 Gew.-% eine oder mehrere erfindungsgemäße Acrylphosphonsäuren,
(b) 0,01 bis 5 Gew.-% und bevorzugt 0,1 bis 2,0 Gew.-% radikalischer Initiator,
(c) 0 bis 80 Gew.-%, bevorzugt 0 bis 60 Gew.-% und besonders bevorzugt 0 bis 50 Gew.-% radikalisch polymerisierbare Comonomere,
(d) 0 bis 95 Gew.-%, bevorzugt 0 bis 80 Gew.-% und besonders bevorzugt 0 bis 70 Gew.-% Lösungsmittel, insbesondere Wasser, Ethanol, Aceton, Essigsäureethylester oder Mischungen davon sowie Mischungen von Wasser mit den genannten organischen Lösungsmitteln,;
(e) 0 bis 90 Gew.-%, besonders bevorzugt in Abhängigkeit von der Anwendung 0 bis 20 Gew.-% (Adhäsiv), 20 bis 60 Gew.-% (Zement) und 60 bis 85 Gew.-% (Füllungskomposit) Füllstoff.

Gemäß einer besonders bevorzugten Ausführungsform sind die erfindungsgemäßen Dentalmaterialien frei von Acrylphosphonsäuren wie sie beispielsweise in der DE 197 46 708 beschrieben werden.

Die Erfindung wird im folgenden anhand von Beispielen näher erläutert.

### Beispiele

### Beispiel 1:

### 1. Stufe: 2-[4-(Dimethoxyphosphoryl)-2-oxa-butyl]-acrylsäure (1)

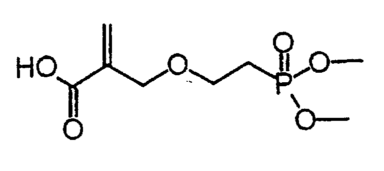

Zu 133 g (0,5 mol) 2-[4-(Dimethoxyphosphoryl)-2-oxa-butyl]-acrylsäureethylester, der durch Umsetzung von 2-Hydroxyethylphosphonsäurediethylester und α-Chlormethylacrylsäureethylester zugänglich ist, (vgl. N. Moszner, F. Zeuner, U.K. Fischer, V. Rheinberger, Macromol. Chem. Phys. 200 (1999) 1062), werden 250 ml (0,5 mol) 2 N KOH so zugetropft, daß die Temperatur zwischen -5 bis 0 °C bleibt. Dann wird die Reaktionsmischung für 2 Stunden bei dieser Temperatur und anschließend noch 2 Stunden bei 25 °C gerührt. Das Produkt wird 3 mal mit je 250 ml Methylenchlorid gewaschen, die zurückbleibende wäßrige Phase wird dann mit konz. Salzsäure auf einen pH-Wert von 1 eingestellt und mit je 250 ml Methylenchlorid 3 mal extrahiert. Nach Trocknen der organischen Extrakte über wasserfreiem Natriumsulfat werden diese am Rotationsverdampfer eingeengt und bei 40 °C im Feinvakuum bis zur Gewichtskonstanz getrocknet. Es verbleiben 106 g (89 % Ausbeute) einer farblosen, viskosen Flüssigkeit, die nach längerem Stehen bei -18 °C fest wird.

| Elementaranalyse: | | | | |
|---|---|---|---|---|
| | C₈H₁₅O₆P: | Ber. | C 40,34 | H 6,35 |
| | (238,18) | Gef. | C 40,24 | H 6,52 |

IR (KBr, cm⁻¹): 2958 (s), 1712 (s), 1634 (m), 1455 (m), 1386 (m), 1218 (s), 1180 (s), 1104 (s), 1033 (s), 956 (m), 824 (m), 702 (w), 651 (m).
¹H-NMR (400 MHz, CDCl₃ ppm): 2,16-2,27 (m, 2H, CH₂P), 3,70-3,80 (m, 7H, CH₃+CH₂CH₂O), 4,21 (s, 2H, C=C-CH₂O), 5,92 und 6,37 (s, 2×1H; CH₂=C), 10,65 (s, 1H, COOH).
¹³C-NMR (100 MHz, CDCl₃, ppm): 22,85 und 24,05 (CH₂P), 51,32 (CH₃), 62,34 (CH₂CH₂O), 66,99 (C=C-CH₂O), 124,18 und 134,83 (C=CH₂), 166,08 (C=O).
³¹P-NMR (161,9 MHz, CDCl₃, ppm): 32,6.

### 2. Stufe: 2-[4-(Dimethoxyphosphoryl)-2-oxabutyl]-acrylsäurediethylamid (2)

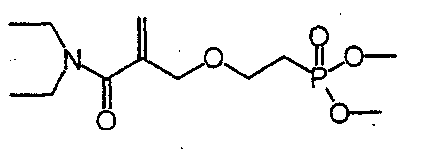

Zu einer Lösung von 35,2 g (148 mmol) **1**, 0,5 g (4,1 mmol) 4-Dimethylaminopyridin (DMAP) und 8 g Hydrochinonmonomethylether (MEHQ), Stabilisator) in 280 ml wasserfreiem Methylenchlorid werden bei -5 °C 10,2 g (140 mmol) Diethylamin so zugetropft, daß die Temperatur 0 °C nicht übersteigt. Anschließend werden 27,0 g (141 mmol) N-(3-Dimethylaminopropyl)-N'-ethylcarbodiimid (EDC) bei der gleichen Temperatur zugegeben. Nach weiterem Rühren bei Raumtemperatur wird der Reaktionsansatz nach 20 Stunden mit 420 ml Methylenchlorid verdünnt und jeweils 2 mal erst mit 2-N NaOH und dann mit 2 N HCl extrahiert. Schließlich wäscht man mit gesättigter NaHCO₃-Lösung und gesättigter NaCl-Lösung. Nach dem Trocknen der Methylenchlorid-Phase über wasserfreiem Na₂SO₄ engt man am Rotationsverdampfer ein und trocknet den Rückstand bei 40 °C und 0,2 mbar bis zur Gewichtskonstanz. Die Feinvakuumdestillation des erhaltenen Rohproduktes ergab bei Kp. 139 °C (0,08 mbar) 21,2 g (52 % Ausbeute) eines hellgelben Öls.

| Elementaranalyse: | | | | | |
|---|---|---|---|---|---|
| | C₁₂H₂₄NO₅P: | Ber. | C 49,14 | H 8,25 | N 4,78 |
| | (293,30) | Gef. | C 47,77 | H 8,22 | N 5,02 |

IR (KBr, cm⁻¹): 3478 (b), 2972 (m), 1644 (s), 1620 (s), 1462 (s), 1252 (s), 1101 (s), 1032 (s), 947 (m), 821 (s).
¹H-NMR (400 MHz, CDCl₃ ppm): 1,19 (t, 6H, CH₃CH₂N), 2,08-2,16 (m, 2H, PCH₂CH₂O), 3,43 (q, 4H, CH₃CH₂N), 3,68-3,79 (m, 8H, OCH₃ und PCH₂CH₂O), 4,18 (s, 2H, OCH₂C=), 5,19 und 5,39 (s, 2×1H; CH₂=).
¹³C-NMR (100 MHz, CDCl₃, ppm): 14,36 und 15,06 (CH₃CH₂N), 25,28 und 26,68 (PCH₂CH₂O), 38,89 und 42,80 (CH₃CH₂N), 52,32 (OCH₃), 64,63 (PCH₂CH₂O), 71,64 (OCH₂C=), 114,57 (CH₂=), 142,22 (C=CH₂), 169,81 (C=O).
³¹P-NMR (161,9 MHz, CDCl₃, ppm): 31,26.

### 3. Stufe: 2-[4-(Dihydroxyphosphoryl)-2-oxabutyl]-acrylsäurediethylamid (3)

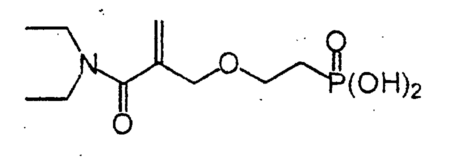

Zu 13,5 g (51 mmol) der Verbindung **2** werden 17 g (111 mmol) Trimethylsilylbromid langsam zugetropft und die Mischung anschließend 2 Stunden bei 40 °C gerührt. Danach engt man erst im Wasserstrahlvakuum und dann im Feinvakuum (0,2 mbar) ein, gibt 70 ml absolutes Methanol hinzu und rührt über Nacht bei Raumtemperatur. Die leicht gelbliche, klare Lösung wird am Rotationsverdampfer eingeengt und im Feinvakuum (0,2 mbar) bei 40 °C bis zu Gewichtskonstanz nachgetrocknet. Es bleiben 13,5 g (100 % Ausbeute) eines hellgelben Öls als Produkt zurück, das eine HPLC-Reinheit von 98,1 % aufweist.
IR (Film, cm⁻¹): 2975 (s), 2936 (s), 2877 (s), 1570 (s), 1489 (s), 1460 (m), 1317 (m), 1215 (s), 1137 (s), 1010 (s), 944 (s), 790 (w).
¹H-NMR (400 MHz, CDCl₃ ppm): 1,15 (t, 6H, CH₃CH₂N), 2,06-2,15 (m, 2H, PCH₂CH₂O), 3,40-3,45 (m, 4H, CH₃CH₂N), 3,73-3,80 (m, 2H, PCH₂CH₂O) 4,17 (s, 2H, OCH₂C=), 5,18 und 5,38 (s, 2×1H; CH₂=), 11,90 (s, 2H, OH).
¹³C-NMR (100 MHz, CDCl₃, ppm): 11,71 und 13,27 (CH₃CH₂N), 26,42 und 27,80 (PCH₂CH₂O), 38,56 und 42,39 (CH₃CH₂N), 64,20 (PCH₂CH₂O), 70,50 (OCH₂C=), 115,05 (CH₂=), 140,30 (C=CH₂), 170,10 (C=O).
³¹P-NMR (161,9 MHz, CDCl₃, ppm): 28,30.

### Beispiel 2:

### 1. Stufe: 2-[4-(Dimethoxyphosphoryl)-2-oxabutyl]-acrylnitril (4)

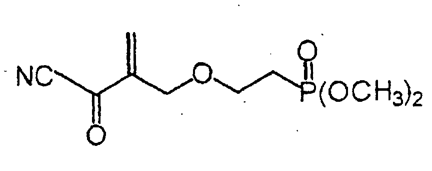

Zu einer Lösung von 130,9 g (850 mmol) 2-Hydroxyethylphosphonsäuredimethylester, 85,9 g (850 mmol) Triethylamin (TEA) und 40 mg Phenothiazin (Stabilisator) in 1 l THF werden bei Raumtemperatur 86,3 g (850 mmol) 2-(Chlormethyl)-acrylnitril zugetropft.

Anschließend wird der Ansatz 6 Stunden bei 65 °C unter Rückfluß erhitzt. Man gibt dann nochmals nacheinander 9,1 g (90 mmol) TEA und 9,1 g (90 mmol) 2-(Chlormethyl)-acrylnitril hinzu und erwärmt für weitere 24 Stunden unter Rückfluß. Nach dem Abkühlen der Reaktionsmischung wird der gebildete Niederschlag an TEA-Hydrochlorid abfiltriert und 2 mal mit 50 ml THF gewaschen. Nach dem Einengen der THF-Phasen am Rotationsverdampfer wird das zurückbleibende Rohprodukt in 650 ml Methylenchlorid aufgenommen und 2 mal mit Wasser gewaschen. Nach Trocknen über wasserfreiem Natriumsulfat wird am Rotationsverdampfer im Vakuum (50 mbar) eingeengt und die zurückbleibende Flüssigkeit im Feinvakuum fraktioniert destilliert. Es ergeben sich bei Kp. 150 °C (0,05 mbar) 101,0 g (54 % Ausbeute) einer farblosen Flüssigkeit.

| Elementaranalyse: | | | | |
|---|---|---|---|---|
| C₈H₁₄NO₄P: | Ber. | C 43,84 | H 6,44 | N 6,39 |
| (219,16) | Gef. | C 43,11 | H 6,98 | N 6,29 |

IR (Film, cm⁻¹): 2956 (m), 2227 (m), 1626 (w), 1464 (m), 1397 (m), 1250 (s), 1107 (s), 1032 (s), 944 (s), 821 (s).
¹H-NMR (400 MHz, CDCl₃ ppm): 2,07-2,19 (m, 2H, PCH₂CH₂O), 3,71-3,83 (m, 8H, OCH₃ und PCH₂CH₂O) 4,11 (s, 2H, OCH₂C=), 6,06 (s, 2H; CH₂=).
¹³C-NMR (100 MHz, CDCl₃, ppm): 25,01 und 26,41 (PCH₂CH₂O), 52,71 (OCH₃), 64,58 (PCH₂CH₂O), 70,03 (OCH₂C=), 117,18 (CN), 120,40 (C=CH₂), 132,82 (CH₂=).
³¹P-NMR (161,9 MHz, CDCl₃, ppm): 30,73.

### 2. Stufe: 2-[4-(Dihydroxyphosphoryl)-2-oxabutyl]-acrylnitril (5)

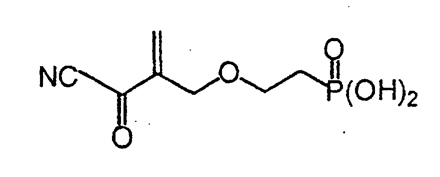

Analog zur Herstellung von **3** wurden 55,0 g (250 mmol) **5** mit 84,2 g (550 mmol) Trimethylsilylbromid umgesetzt und aufgearbeitet. Es ergaben sich 47,5 g (99 % Ausbeute) eines dunklen, wachsartigen Feststoffs.

| Elementaranalyse: | | | | | |
|---|---|---|---|---|---|
| | C₆H₁₀NO₄P: | Ber. | C 37,71 | H 5,27 | N 7,33 |
| | (191,12) | Gef. | C 36,15 | H 5,60 | N 7,26 |

IR (Film, cm⁻¹): 2884 (m), 2228 (m), 1722 (w), 1456 (w), 1398 (w), 1103 (s), 1009 (s), 954 (s).
¹H-NMR (400 MHz. CDCl₃ ppm): 2,16-2,23 (m, 2H, PCH₂CH₂O), 3,67-3,81 (m, 2H, PCH₂-CH₂O) 4,10 (s, 2H, OCH₂C=), 6,07 (s, 2H; CH₂=), 10,93 (s, 2H, OH).
¹³C-NMR (100 MHz, CDCl₃, ppm): 34,06 und 35,96 (PCH₂CH₂O), 72,04 (PCH₂CH₂O), 76,90 (OCH₂C=), 124,28 (CN), 126,66 (C=CH₂), 139,81 (CH₂=).
³¹P-NMR (161,9 MHz, CDCl₃, ppm): 30,71.

### Beispiel 3:

### 1. Stufe: N,N'-Bis-[(6-dimethoxyphosphoryl)-4-oxa-2-methylenhexanoyl]-1,2-diaminoethan (6)

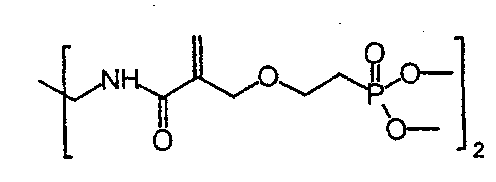

Analog zur Herstellung von **2** wurden eine Lösung von 94 g (395 mmol) **1**, 1,3 g (10,8 mmol) DMAP und 18 mg MEHQ in 750 ml wasserfreiem Methylenchlorid bei -5 °C mit 10,8 g (180 mmol) Ethylendiamin und 69,3 g (360 mmol) EDC umgesetzt. Nach analoger Aufarbeitung des Reaktionsansatzes ergaben sich 26,8 g (30 % Ausbeute) eines gelben Öls.

| Elementaranalyse: | | | | | |
|---|---|---|---|---|---|
| | C₁₈H₃₄N₂O₈P₂: | Ber. | C 43,20 | H 6,85 | N 5,60 |
| | (500,43) | Gef. | C 43,04 | H 6,94 | N 5,52 |

IR (Film, cm⁻¹): 3322 (m), 2954 (m), 1667 (s), 1622 (s), 1538 (s), 1248 (s), 1104 (s), 1032 (s), 951 (w), 823 (m).
¹H-NMR (400 MHz, CDCl₃ ppm): 2,07-2,18 (m, 4H, PCH₂CH₂O), 3,52 (s, 4H, CH₂NH), 3,71-3,79 (m, 16H, OCH₃ und PCH₂CH₂O) 4,20 (s, 4H, OCH₂C=), 5,59 und 6,06 (s, 2×2H; CH₂=), 7,84 (s, 2H, NH).
¹³C-NMR (100 MHz, CDCl₃, ppm): 24,88 und 26,28 (PCH₂CH₂O), 39,57 (CH₂NH), 52,45 (OCH₃), 64,20 (PCH₂CH₂O), 70,79 (OCH₂C=), 123,34 (CH₂=), 139,50 (C=CH₂), 167,20 (C=O).
³¹P-NMR (161,9 MHz, CDCl₃, ppm): 31,74.

### 2. Stufe: N,N'-Bis-[(6-dihydroxyphosphoryl)-4-oxa-2-methylenhexanoyl]-1,2-diaminoethan (7)

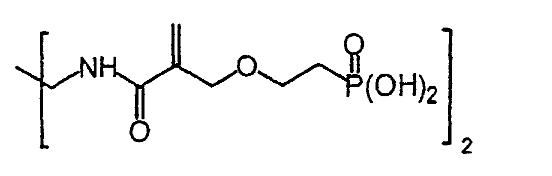

Analog zur Herstellung von **3** wurden 11,3 g (22,6 mmol) **6** mit 15,3 g (100 mmol) Trimethylsilylbromid umgesetzt und aufgearbeitet. Es ergaben sich 10 g (96 % Ausbeute) eines rötlichen Feststoffs.
IR (Film, cm⁻¹): 3347 (s, b), 2880 (s), 2318 (m), 1659 (s), 1622 (s), 1609 (s), 1552 (s), 1438 (m), 1362 (m), 1316 (m), 1095 (s), 1002 (s), 935 (s), 715 (m).
¹H-NMR (400 MHz, CDCl₃ ppm): 1,94-2,02 (m, 4H, PCH₂CH₂O), 3,34 (s, 4H, CH₂NH), 3,50-3,65 (m, 4H, PCH₂CH₂O) 4,10 (s, 4H, OCH₂C=), 4,70 (s, POH+H₂O), 5,64 und 6,23 (2s, 2×2H; CH₂=).
¹³C-NMR (100 MHz, CDCl₃ ppm): 26,95 und 28,29 (PCH₂CH₂O), 39,05 (CH₂NH), 64,66 (PCH₂CH₂O), 70,24 (OCH₂C=), 125,04 (CH₂=), 139,39 (C=CH₂), 170,32 (C=O).
³¹P-NMR (161,9 MHz, CDCl₃, ppm): 26,89.

### Beispiel 4

### Radikalische Homopolymerisation der Monomeren 3 und 5

5,31 g (20,0 mmol) Monomer **3** bzw. 3,82 g (20,0 mmol) Monomer **5** und jeweils 2,0 Mol-% 2,2'-Azobis(isobuttersäureamidin)dihydrochlorid werden mit dest. Wasser auf jeweils auf 10 ml Monomerlösung in einem Schlenkgefäß aufgefüllt. Die Monomerlösungen werden durch mehrfach wiederholtes Einfrieren unter Argon und Auftauen unter Feinvakuum-entgast und anschließend bei 65 °C unter Argon polymerisiert. Während der Polymerisation nimmt die Viskosität der Ausgangslösung deutlich zu. Nach 2 Stunden fällt man die Lösungen in der 10-fachen Menge an Tetrahydrofuran aus und trocknet bis zur Gewichtskonstanz. Der so gravimetrisch ermittelte Monomerumsatz beträgt für Monomer **3** 23,1 % und für Monomer **5** 13,7 %. Der Erfolg der Polymerisation läßt sich auch ¹H-NMR-spektroskopisch bestätigen.

### Beispiel 5

### Untersuchung der hydrolytischen Stabilität der Monomeren 3 und 5

Die Monomeren **3** bzw. **5** werden jeweils in einer 1:1-Mischung aus Wasser und Ethanol gelöst und als 20%-ige Lösung bei 37 °C gelagert. Wochenweise wird das ¹H-NMR-Spektrum der Lösung aufgenommen. Im Untersuchungszeitraum von 12 Wochen ergab sich keine Veränderung des Spektrums von Monomer **3** oder **5**, was dessen hydrolytische Stabilität belegt. Unter analogen Bedingungen wurde das Monomer 2-[4-(Dimethoxyphosphoryl)-2-oxa-butyl]-acrylsäureethylester (Vergleichsmonomer 1; X = COO, Z = Ethyl) untersucht, wobei nach 3 Monaten ¹H-NMR-spektroskopisch eine 20%-ige hydrolytische Abspaltung der Ethoxygruppe nach folgender Gleichung festgestellt werden konnte:

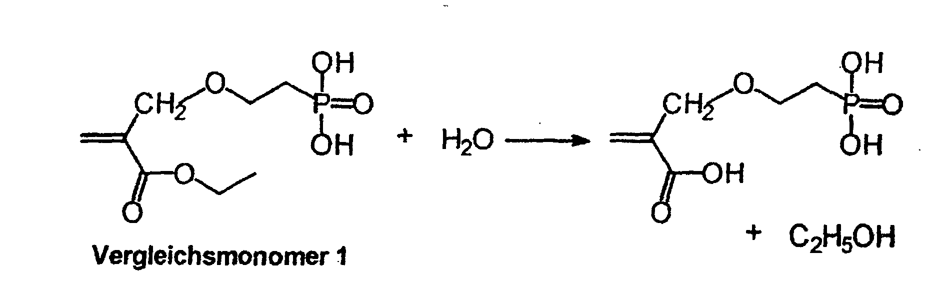

### Beispiel 6

### Untersuchung der Dentinhaftung von Monomer 3

Zur Untersuchung der Dentinhaftung auf Rinderzahndentin wurde ein Adhäsiv folgender Zusammensetzung (Angabe in Masse-%) hergestellt:

| | |
|---|---|
| Monomer **3** | 11,1 % |
| Glycerindimethacrylat | 11,0 % |
| 2-Hydroxyethylmethacrylat | 20,0 % |
| Ethanol | 24,0 % |
| Bis-GMA | 33.1 % |
| Photoinitiator | 0,8 % |

Rinderzähne werden so in Kunststoffzylinder eingebettet, daß sich das Dentin und der Kunststoff in einer Ebene befinden. Nach 15 Sekunden Ätzung mit 37%-iger Phosphorsäure wird gründlich mit Wasser abgespült. Durch die Säureätzung sind die Dentubili geöffnet. Dann wird mit einem Microbrush eine Schicht Adhäsiv obiger Zusammensetzung aufgepinselt, mit dem Luftbläser zur Entfernung des Lösungsmittels kurz verblasen und für 40 Sekunden mit einer Halogenlampe (Astralis 7, Vivadent) belichtet. Auf die Adhäsivschicht polymerisiert man einen Kompositzylinder aus Tetric® Ceram (Vivadent) in zwei Schichten von je 1-2 mm auf. Anschließend werden die Prüfkörper 24 h bei 37 °C in Wasser gelagert und dann die Scherhaftfestigkeit-bestimmt. Es wurde ein Wert von 23,2 MPa ermittelt.

### Beispiel 7

### Untersuchung der Schmelzhaftung von Monomer 5

Zur Untersuchung der Schmelzhaftung auf Rinderzahnschmelz wurde ein Adhäsiv folgender Zusammensetzung (Angabe in Masse-%) hergestellt:

| | |
|---|---|
| Monomer **5** | 11,0 % |
| Glycerindimethacrylat | 10,0 % |
| 2-Hydroxyethylmethacrylat | 20,0 % |
| Ethanol | 25,5 % |
| Bis-GMA | 32.7 % |
| Photoinitiator | 0,8 % |

Rinderzähne werden so in Kunststoffzylinder eingebettet, daß sich die Schmelzzone und der Kunststoff in einer Ebene befinden. Nach 15 s Ätzung mit 37%-iger Phosphorsäure wird gründlich mit Wasser abgespült. Dann wird mit einem Microbrush eine Schicht Adhäsiv obiger Zusammensetzung aufgepinselt, mit dem Luftbläser zur Entfernung des Lösungsmittels kurz verblasen und für 40 Sekunden mit einer Halogenlampe (Astralis 7, Vivadent) belichtet. Auf die Adhäsivschicht polymerisiert man einen Kompositzylinder aus Tetric® Ceram (Vivadent) in zwei Schichten von je 1-2 mm auf. Anschließend werden die Prüfkörper 24 h bei 37 °C in Wasser gelagert und dann die Scherhaftfestigkeit bestimmt. Es wurde ein Wert von 15,5 MPa ermittelt.

### Beispiel 8

### Untersuchung der Löslichkeit der Monomeren 3 und 5

Es wurde die Löslichkeit der Monomeren 3 und 5 und von einem Vergleichsmonomer (Vergleichsmonomer 2; X = COO, Z = H) in Wasser, Ethanol, Aceton, Methylenchlorid und Ethylacetat untersucht. Die Ergebnisse sind nachfolgend zusammengestellt.

**Tabelle 1**

| Vergleich der Löslichkeiten von Carbonsäure-, Carbonsäureamid- und Carbonsäurenitrilderivaten von Acrylphosphonsäure | | | |
|---|---|---|---|
| ↓Löslichkeit/Monomer→ | 3 | 5 | Vergleichsmonomer 2 |
| Wasser | ++ | ++ | ++ |
| Ethanol | ++ | ++ | 0 |
| Methylenchlorid | ++ | ++ | 0 |
| Aceton | ++ | ++ | 0 |
| Ethylacetat | ++ | ++ | 0 |

Löslichkeit: ++: sehr gut (> 20 Gew.-%), +: gut (10 - 20 Gew.-%), 0: praktisch unlöslich (< 1 Gew.-%)

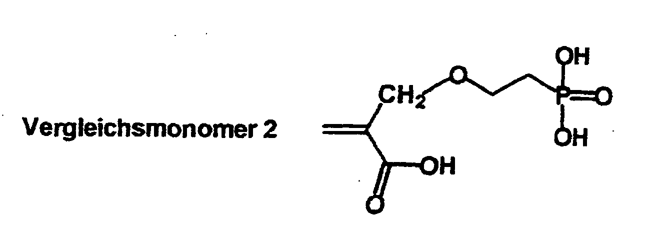

## Patentansprüche

1. Acrylphosphonsäure der allgemeinen Formel (I), Stereoisomere davon oder Mischungen von diesen 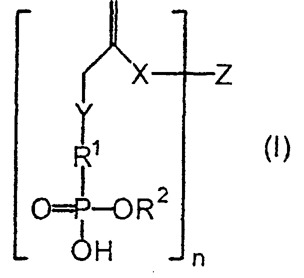 worin R¹, R², R³, X, Y, Z und n die folgenden Bedeutungen haben:
R¹ = ein geradkettiger oder verzweigter C₁- bis C₁₀-Alkylenoder C₆- bis C₁₄-Arylen-Rest;
R² = Wasserstoff, ein geradkettiger oder verzweigter C₁- bis C₁₀-Alkyl- oder C6- bis C₁₀-Arylrest;
Y = Sauerstoff, Schwefel, C₁- bis C₈-Alkylen oder entfällt;
n = 1, 2, 3, 4, oder 5;
wobei
X = CN, n = 1 und Z = entfällt oder
X = CONR³ mit
R³ = Wasserstoff, ein geradkettiger oder verzweigter C₁- bis C₁₀-Alkylrest oder ein C₆- bis C₁₀-Aryl-Rest;
mit der Maßgabe daß
für
n = 1
Z = Wasserstoff oder ein geradkettiger oder verzweigter C₁- bis C₁₀-Alkylrest oder ein Phenylrest; und
für
n = 2 bis 5
Z = ein n-fach mit der in Klammern stehenden Struktur der Formel (I) substituierter, aliphatischer, aromatischer oder araliphatischer, geradkettiger oder verzweigter Kohlenwasserstoffrest mit 1 bis 14 Kohlenstoffatomen, wobei Z und R³ auch Teil eines gemeinsamen Ringes sein können, und
wobei die einzelnen Reste substituiert oder unsubstituiert sein können.

2. Acrylphosphonsäure nach Anspruch 1, **dadurch gekennzeichnet, daß** die Variablen der Formel (I) unabhängig voneinander die folgenden Bedeutungen haben:
R¹ = ein geradkettiger oder verzweigter C₁- bis C₅-Alkylenrest oder Phenylen;
R² = Wasserstoff oder ein geradkettiger C₁- bis C₃-Alkylrest;
Y = Sauerstoff oder entfällt;
X = CN oder CONR³ mit
R³ = Wasserstoff, ein geradkettiger C₁- bis C₆-Alkylrest, ein Phenylrest oder zusammen mit Z Teil eines sechsgliedrigen Rings;
n = 1 oder 2; und
Z = Wasserstoff oder ein geradkettiger oder verzweigter C₁bis C₁₀-Alkylrest, ein Phenylrest oder zusammen mit R³ Teil eines sechsgliedrigen Rings (für n = 1); oder
Z = ein geradkettiger C₁- bis C₁₀-Alkylenrest oder zusammen mit R³ Teil eines sechsgliedrigen Rings (für n = 2).

3. Acrylphosphonsäure nach Anspruch 2, **dadurch gekennzeichnet, daß** die Variablen der Formel (I) unabhängig voneinander die folgenden Bedeutungen haben:
R¹ = ein geradkettiger C₁- bis C₄-Alkylenrest;
R² = Wasserstoff oder ein Methylrest;
Y = Sauerstoff;
X = CONR³;
R³ = Wasserstoff oder ein geradkettiger C₁- bis C₅-Alkylrest; und
Z = Wasserstoff oder ein geradkettiger C₁- bis C₆-Alkylrest (für n = 1); oder
Z = ein geradkettiger C₁- bis C₅-Alkylenrest (für n = 2).

4. Acrylphosphonsäure nach einem der Ansprüche 1 bis 3, **dadurch gekennzeichnet, daß** die Reste R¹, R², R³ und/oder Y unsubstituiert sind.

5. Acrylphosphonsäure nach einem der Ansprüche 1 bis 4, **dadurch gekennzeichnet, daß** der Rest Z unsubstituiert oder durch =O, =S, =NR² oder -NR³-CO-C(=CH₂)CH₂-Y-R¹-PO(OH)₂ substituiert ist.

6. Verwendung der Acrylphosphonsäure gemäß Anspruch 1 bis 5 als Bestandteil eines Adhäsivs, eines Polymeren, eines Komposits, eines Zements, eines Formkörpers und insbesondere eines Dentalmaterials.

7. Verwendung nach Anspruch 6, **dadurch gekennzeichnet, daß** das Dentalmaterial ein Dentaladhäsiv, ein Befestigungszement oder ein Füllungskomposit ist.

8. Verwendung nach Anspruch 6 oder 7, **dadurch gekennzeichnet, daß** die Acrylphosphonsäure in zumindest teilweise polymerisierter Form vorliegt.

9. Dentalmaterial, **dadurch gekennzeichnet, daß** es eine Acrylphosphonsäure gemäß Anspruch 1 bis 5 enthält.

10. Dentalmaterial nach Anspruch 9, **dadurch gekennzeichnet, daß** es die Acrylphosphonsäure in zumindest teilweise polymerisierter Form enthält.

11. Polymere und Copolymere, **dadurch gekennzeichnet, daß** sie durch Polymerisation oder Copolymerisation einer Acrylphosphonsäure gemäß einem der Ansprüche 1 bis 5 erhältlich sind.

## Claims

1. Acrylophosphonic acid of the general formula (I), stereoisomers thereof or mixtures of these 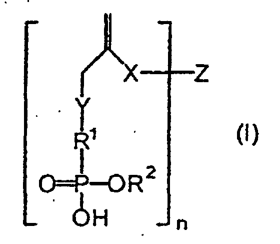 in which R¹, R², R³, X, Y, Z and n have the following meanings:
R¹ = a linear or branched C₁ to C₁₀ alkylene or C₆ to C₁₄arylene radical;
R² = hydrogen-, a linear or branched C₁ to C₁₀ alkyl or C₆ to C₁₀ aryl radical;
Y = oxygen, sulphur, C₁ to C₈ alkylene or is absent;
n = 1, 2, 3, 4 or 5;
where
X = CN, n = 1 and Z is absent or
X = CONR³ with
R³ = hydrogen, a linear or branched C₁ to C₁₀ alkyl radical, or a C₆ to C₁₀ aryl radical;
provided that
for n = 1
Z = hydrogen or a linear or branched C₁ to C₁₀ alkyl radical, or a phenyl radical; and
for n = 2 to 5
Z = an aliphatic, aromatic, or araliphatic, linear or branched hydrocarbon radical with 1 to 14 carbon atoms, substituted n times with the structure of formula (I) in brackets, where Z and R³ may also be part of a common ring, and where
the individual radicals may be substituted or unsubstituted.

2. Acrylophosphonic acid according to claim 1, **characterized in that** the variables of formula (I) have the following meanings independently of each other:
R¹ = a linear or branched C₁ to C₅ alkylene radical or phenylene;
R² = hydrogen or a linear C₁ to C₃ alkyl radical;
Y = oxygen or is absent;
X = CN or CONR³ with
R³ = hydrogen, a linear C₁ to C₆ alkyl radical, a phenyl radical or together with Z part of a six-membered ring;
n = 1 or 2; and
Z = hydrogen or a linear or branched C₁ to C₁₀ alkyl radical, a phenyl radical or together with R³ part of a six-membered ring (for n = 1); or
Z = a linear C₁ to C₁₀ alkylene radical or together with R³ part of a six-membered ring (for n = 2).

3. Acrylophosphonic acid according to claim 2, **characterized in that** the variables of formula (I) have the following meanings independently of each other:
R¹ = a linear C₁ to C₄ alkylene radical;
R² = hydrogen or a methyl radical;
Y = oxygen;
X = CONR³;
R³ = hydrogen or a linear C₁ to C₃ alkyl radical; and
Z = hydrogen or a linear C₁ to C₆ alkyl radical (for n=1); or
Z = a linear C₁ to C₅ alkylene radical (for n = 2).

4. Acrylophosphonic acid according to one of claims 1 to 3, **characterized in that** the radicals R¹, R², R³ and/or Y are unsubstituted.

5. Acrylophosphonic acid according to one of claims 1 to 4, **characterized in that** the radical Z is unsubstituted or is substituted by =O, =S, =NR² or -NR³-CO-C(=CH₂)CH₂-Y-R¹-PO(OH)₂.

6. Use of the acrylophosphonic acid according to claims 1 to 5 as a component of an adhesive, of a polymer, of a composite, of a cement, of a moulded article and in particular of a dental material.

7. Use according to claim 6, **characterized in that** the dental material is a dental adhesive, a fixing cement or a filling composite.

8. Use according to claim 6 or 7, **characterized in that** the acrylophosphonic acid is present in at least partially polymerized form.

9. Dental material, **characterized in that** it contains an acrylophosphonic acid according to claims 1 to 5.

10. Dental material according to claim 9, **characterized in that** it contains the acrylophosphonic acid in at least partially polymerized form.

11. Polymers and copolymers, **characterized in that** they can be obtained by polymerization or copolymerization of an acrylophosphonic acid according to one of claims 1 to 5.

## Revendications

1. Acides acrylphosphoniques de la formule générale (I), stéréoisomères de ceux-ci ou mélanges de ceux-ci : 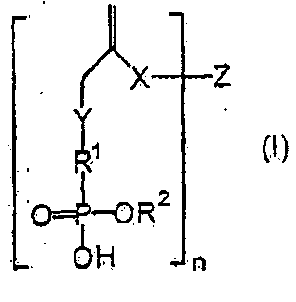 où R¹, R², R³, X, Y, Z et n ont les significations suivantes :
R¹ = un résidu linéaire ou ramifié alkylène en C₁ à C₁₀ ou arylène en C₆ à C₁₄ ;
R² = hydrogène, un résidu linéaire ou ramifié alkyle en C₁ à C₁₀ ou aryle en C₆ à C₁₀ ;
Y = oxygène, soufre, alkylène en C₁ à C₈ ou est absent ;
n = 1, 2, 3, 4 ou 5 ;
où
X = CN, n = 1 et Z est absent, ou
X = CONR³ avec
R³ = hydrogène, un résidu alkyle en C₁ à C₁₀ linéaire ou ramifié ou un résidu aryle en C₆ à C₁₀ ;
avec la condition que
pour n = 1,
Z = hydrogène ou un résidu alkyle en C₁ à C₁₀ linéaire ou ramifié ou un résidu phényle, et
pour n = 2 à 5
Z = un résidu hydrocarbure ayant 1 à 14 atomes de carbone, aliphatique, aromatique ou araliphatique, linéaire ou ramifié, substitué n fois avec la structure de la formule (I) se trouvant entre parenthèse, où Z et R³ peuvent être également, une partie d'un cycle commun, et
où les résidus individuels peuvent être substitués ou non substitués.

2. Acides acrylphosphoniques selon la revendication 1, **caractérisés en ce que** les variables de la formule (I) ont indépendamment l'une de l'autre, les significations suivantes :
R¹ = un résidu alkylène en C₁ à C₅ linéaire ou ramifié ou phénylène ;
R² = hydrogène ou un résidu alkyle en C₁ à C₃ linéaire ;
Y = oxygène ou est absent ;
X = CN ou CONR³ avec
R³ = hydrogène, un résidu alkyle en C₁ à C₆ linéaire, un résidu phényle ou avec Z, une partie d'un cycle à 6 membres ;
n = 1 ou 2 ;
Z = hydrogène ou un résidu alkyle en C₁ à C₁₀ linéaire ou ramifié, un résidu phényle ou avec R³, une partie d'un cycle à 6 membres (pour n = 1), ou
Z = un résidu alkylène en C₁ à C₁₀ linéaire ou avec R³, une partie d'un cycle à 6 membres (pour n = 2).

3. Acides acrylphosphoniques selon la revendication 2, **caractérisé en ce que** les variables de la formule (I) ont indépendamment l'une de l'autre, les significations suivantes :
R¹ = un résidu alkylène en C₁ à C₄ linéaire ;
R² = hydrogène ou le résidu méthyle ;
Y = oxygène ;
X = CONR³ ;
R³ = hydrogène ou un résidu alkyle en C₁ à C₅ linéaire ;
Z = hydrogène ou un résidu alkyle en C₁ à C₆ linéaire (pour n = 1), et
Z = un résidu alkylène en C₁ à C₅ linéaire (pour n = 2).

4. Acides acrylphosphoniques selon l'une quelconque des revendications 1 à 3, **caractérisée en ce que** les résidus R¹, R², R³ et/ou Y sont non substitués.

5. Acides acrylphosphoniques selon l'une quelconque des revendications 1 à 4, **caractérisée en ce que** le résidu Z est non substitué ou est substitué par =O, =S, =NR² ou -NR³-CO-C(=CH₂)CH₂-Y-R¹-PO(OH)₂.

6. Utilisation de l'acide acrylphosphonique selon l'une quelconque des revendications 1 à 5, comme constituant d'un adhésif, d'un polymère, d'un composite, d'un ciment, d'un article moulé et en particulier, d'un matériau dentaire.

7. Utilisation selon la revendication 6, **caractérisée en ce que** le matériau dentaire est un adhésif dentaire, un ciment de renforcement ou un composite de charge.

8. Utilisation selon la revendication 6 ou 7, **caractérisée en ce que** l'acide acrylphosphonique est présent au moins partiellement, sous forme polymérisée.

9. Matériau dentaire **caractérisé en ce qu'**il contient l'acide acrylphosphonique selon les revendications 1 à 5.

10. Matériau dentaire selon la revendication 9, **caractérisé en ce qu'**il contient l'acide acrylphosphonique sous forme au moins partiellement polymérisée.

11. Polymères et copolymères, **caractérisés en ce qu'**ils sont obtenus par polymérisation ou copolymérisation d'un acide acrylphosphonique selon l'une quelconque des revendications 1 à 5.
